# EUROPÄISCHE PATENTSCHRIFT

(11) **EP 0 821 965 B1**
(45) Veröffentlichungstag und Bekanntmachung des Hinweises auf die Patenterteilung: **20.10.2004**
(21) Anmeldenummer: 97112660.2
(22) Anmeldetag: 24.07.1997
(51) Int. Cl.: A61K 31/78

(54) **Verwendung von (Meth)arcylsäure-Maleinsäure-Copolymeren zur Verbesserung der Permeabilität der Schleimhaut**
Use of copolymers of (meth)accrylic and maleic acid for the manufacture of a medicament for enhancing the permeability of the mucose
Utilisation de copolymères de l'acide (meth)acrylique et de l'acide maléique pour la fabrication d'un médicament pour améliorer la perméabilité des muqueuses

(30) Priorität: 01.08.1996 DE 19631085
(43) Veröffentlichungstag der Anmeldung: 04.02.1998
(73) Patentinhaber: BASF AKTIENGESELLSCHAFT, 67056 Ludwigshafen (DE)
(72) Erfinder: Luessen, Henrik L., 56579 Rengsdorf (DE); Borchard, Gerrit, 66123 Saarbrücken (DE); de Boer, Albertus G., 2341 NG Oegstgeest (NL); Junginger, Hans E., 2231 XA Rijnsburg (NL); Kolter, Karl, Dr., 67117 Limburgerhof (DE); Schehlmann, Volker, Dr., 67354 Römerberg (DE); Sanner, Axel, Dr., 67227 Frankenthal (DE)

(56) Entgegenhaltungen:
- WO-A-93/23011
- US-A- 3 551 556
- ACHAR L ET AL: "PREPARATION, CHARACTERIZATION AND MUCOADHESIVE INTERACTIONS OF POLY(METHACRYLIC ACID) COPOLYMERS WITH RAT MUCOSA" JOURNAL OF CONTROLLED RELEASE,NL,ELSEVIER SCIENCE PUBLISHERS B.V. AMSTERDAM, Bd. 31, Nr. 3, 1. Oktober 1994 (1994-10-01), Seiten 271-276, XP000469855 ISSN: 0168-3659
- SARAYDIN D ET AL: "Preparation of acrylamide/maleic acid hydrogels and their biocompatibility with some biochemical parameters of human serum" RADIATION PHYSICS AND CHEMISTRY,NL,ELSEVIER SCIENCE PUBLISHERS BV., AMSTERDAM, Bd. 46, Nr. 46, 12. September 1995 (1995-09-12), Seiten 1049-1052, XP004051235 ISSN: 0969-806X

## Beschreibung

Die vorliegende Erfindung betrifft die Verwendung von (Meth)acrylsäure-Maleinsäure-Copolymeren zur Verbesserung der Permeabilität der Schleimhaut.

Für den parazellulären Transport hydrophiler Wirkstoffe, insbesondere solcher mit höher molekularen Strukturen, bildet das Epithelgewebe eine wichtige Permeationsschranke. Sogenannte tight junctions" (interzelluläre Verbindungsstellen zwischen benachbarten Epithelzellen, in deren Bereich die Plasma-Membranen unmittelbar aneinanderliegen) gewährleisten, daß das interne Milieu eines Organs vom externen abgeschlossen ist. Die passive parazelluläre Permeabilität dieser Epithelzellen wird bestimmt durch die "Engmaschigkeit" der interzellulären Kontaktpunkte. Eine Aufweitung der "tight junctions" führt zu einer verbesserten Absorption und somit zu einer höheren Bioverfügbarkeit von Wirkstoffen.

Es hat daher in der Vergangenheit nicht an Versuchen gefehlt, Methoden zur Öffnung der interzellulären Kontaktstellen zu entwickeln. Hierbei zeigte sich, daß der Einsatz sowohl oberflächenaktiver Reagentien als auch Ca²⁺- chelatisierender Substanzen vielversprechende Ansatzpunkte lieferte.

Gemäß J. Controlled Rel., 29 (1994) 253, besteht bei Anwendung von oberflächenaktiven Substanzen jedoch die Gefahr einer Zell-Lyse und damit verbundenen toxischen Nebenwirkungen.

Zahlreiche Publikationen, u.a. in J. Controlled Rel., 36 (1995) 25; Chem. Pharm. Bull. 33 (1985) 4600; The Journal of Cell Biology, 87 (1980) 736 und Int. J. Pharm., 90 (1993) 229 beschreiben den Einfluß von EDTA bzw. EGTA auf die Permeabilität unterschiedlicher Zellsysteme, z.B. von Caco-2-Zellen. Danach kann die Anwesenheit von Ca²⁺-chelatisierenden Substanzen zu einer schnellen aber häufig nicht reversiblen Öffnung der tight junctions führen. Im Falle von EDTA benötigt man außerdem relativ hohe Konzentrationen, um bei neutralem pH-Wert einen Effekt (Reduktion des transepithelialen Widerstandes) zu beobachten. Daneben besteht bei Komplexbildnern mit einem Molekulargewicht ≤ 20 kDa die Gefahr, daß sie systematisch absorbiert werden und so zu ungewünschten toxischen Nebenwirkungen führen können.

In J. Controlled Rel., 29 (1994) 329 konnte gezeigt werden, daß nicht absorbierbare hochmolekulare Verbindungen auf Basis quervernetzter Polyacrylate wie Polycarbophil (Noveon® AA1, B.F. Goodrich) ebenfalls in der Lage sind, tight junctions zu öffnen. Aufgrund ihres extrem hohen Molekulargewichtes (> 1.000 kDa) und ihrer bereits bei niedrigen Konzentrationen (≥ 0.5 Gew.-%) hohen Viskosität ergeben sich anwendungstechnische Nachteile.

Weiter ist bekannt, daß Polymere mit bioadhäsiven Eigenschaften die Bioverfügbarkeit von Wirkstoffen verbessern können. So wird beispielsweise in EP-A-587 047 die Verwendung von Copolymeren aus (Meth)acrylaten mit verschiedenen Carbonsäuren für pharmazeutische Zubereitungen auf Gestagen-Basis beschrieben.

Die Bioverfügbarkeit schwerlöslicher Wirkstoffe läßt sich nach EP-B-410 422 dadurch erhöhen, daß sie nach der Formulierung durch den Einfluß von (Meth)acrylsäure/(Meth)acrylat-Copolymeren amorph vorliegen und somit besser löslich sind.

Es war nun Aufgabe der Erfindung, Polymere mit chelatisierenden Eigenschaften zu finden, die die Permeabilität von Epithelzellen erhöhen, ohne dabei die oben genannten anwendungstechnischen Nachteile aufzuweisen oder Toxizitätsprobleme zu verursachen.

Demgemäß wurde nun gefunden, daß durch Verwendung von (Meth)acrylsäure-Maleinsäure-Copolymeren, insbesondere solchen, enthaltend a) 10 bis 90 mol-% (Meth)acrylsäure, b) 90 bis 10 mol-% Maleinsäure und c) 0 bis 40 mol-% weiterer Monomere eine Verbesserung der Permeabilität der Schleimhaut erzielt wird.

Ausgangsmonomere a) sind Acrylsäure und/oder Methacrylsäure, ihre Anhydride, ihre Salze oder Mischungen aus den beiden genannten Carbonsäuren, Anhydriden und Salzen. Monomer b) kann Maleinsäure, ihr Salz oder Maleinsäureanhydrid sein.

Die Monomeren a) und b) können auch teilweise in Form ihrer Salze vorliegend eingebaut sein. Das kann beispielsweise durch Zusatz einer Base vor, während oder nach der Polymerisation erreicht werden. Liegen die Monomere in Form ihrer Salze vor, so sind die Erdalkali-, Alkali- oder Ammoniumsalze oder die Salze organischer Amine bevorzugt, besonders bevorzugt sind die Alkali- oder Ammoniumsalze.

Monomere der Gruppe c) sind Vinylsulfonsäure und/oder Hydroxy-C₂bis C₆-alkylester der Acryl- bzw. Methacrylsäure und/oder Acrylester oder Methacrylester von gesättigten, linearen oder verzweigten C₁-bis C₄₀-Alkoholen. Beispiele sind Methyl-, Ethyl-, n-Propyl-, i-Propyl-, n-Butyl-, i-Butyl-, tert. Butyl-, n-Hexyl-, n-Octyl-, i-Nonyl-, n-Decyl-, n-Dodecyl-, n-Tetradecyl-, n-Hexadecyl-, n-Heptadecyl-, n-Octadecyl-, n-Nonadecyl-, n-Eicosyl-, n-Docosyl-, n-Tetracosyl-, 2-Ethylhexyl-, i-Bornylacrylat oder Cyclohexylacrylat bzw. die entsprechenden Methacrylate. Bevorzugt eingesetzt werden C₁-, C₂- und/oder C₆-C₃₀-Alkylacrylate oder -methacrylate. Besonders bevorzugt werden C₈-C₂₂-Alkylacrylate oder -methacrylate eingesetzt.

Die Hydroxyalkylestergruppen der Monomeren c) leiten sich beispielsweise von Alkandiolen wie Ethandiol-1,2, Propandiol-1,3, und -1,2 sowie deren technische Gemische, Butandiol-1,4,-1,3 und - 2,3 sowie ihrer Gemische ab. Als Beispiele seien genannt Hydroxyethylacrylat, Hydroxyethylmethacrylat, Hydroxypropylacrylate, Hydroxypropylmethacrylate, Hydroxybutylacrylate, Hydroxybutylmethacrylate. Die bevorzugten Hydroxyalkylester c) sind Hydroxyethylacrylat, Hydroxypropylacrylate und Hydroxybutylacrylate. Die besonders bevorzugten Hydroxyalkylester c) sind die Hydroxypropylacrylate, wobei von besonderer technischer Bedeutung die Isomerengemische aus 2-Hydroxy-1-propylacrylat und 1-Hydroxy-2-propylacrylat sind, die durch Umsetzung von Acrylsäure mit Propylenoxid hergestellt werden.

Das Molverhältnis (Meth)acrylsäure:Maleinsäure kann im erfindungsgemäß zu verwendeten Copolymer breit variiert weren. Beispielsweise liegt es im Bereich von 90:10 bis 10:90, bevorzugt von 70:30 bis 30:70. Bei Anwesenheit des Monomeren c) liegt eine vorteilhafte Monomerenverteilung im Bereich von 5 bis 40, insbesondere 10 bis 35 mol-% c) und 95 bis 60 mol-% a) und b), wobei die Verhältnisse a) und b) wie vorstehend genannt gewählt werden können.

Die Herstellung der Copolymere erfolgt in an sich bekannter Weise durch Suspensionspolymerisation, Fällungspolymerisation oder Lösungspolymerisation, wobei die Lösungspolymerisation in wäßriger Lösung eine bevorzugte Methode darstellt. Beispiele für solche Polymerisate geben die Dokumente EP-A-0 168 547, EP-A-0 075 820 oder EP-A-0 349 810 wieder. Auf diese Weise werden Copolymere mit einem mittleren Molekulargewicht (Gewichtsmittel) von 500 bis 1 Million erhalten. Für die erfindungsgemäße Verwendung sind Copolymere mit einem Molekulargewicht von 5000 bis 500000 insbesondere 10000 bis 300000 besonders geeignet.

Üblicherweise werden die Copolymere aus a), b) und ggf. c) in Form von pharmazeutischen Zubereitungen zusammen mit dem Wirkstoff verabreicht. Als pharmazeutische Darreichungsform kommen Tabletten, Extrudate, Granulate, Pellets, Pulver, Kapseln, Suppositorien, Salben, Lösungen, Suspensionen oder Emulsionen in Betracht, wobei die Verabreichung je nach Anwendungsgebiet oral, sublingual, buccal, rectal, pulmonal, nasal oder über die Augenschleimhaut erfolgen kann. In diesen Darreichungsformen ist der Anteil an erfindungsgemäß zu verwendendem Copolymer im allgemeinen größer als 10 Gew.-%, vorzugsweise größer 30, besonders bevorzugt größer 50 Gew.-%, jeweils bezogen auf die Gesamtmasse der Darreichungsform. Es ist aber auch möglich, zunächst die Schleimhaut, z.B. Magen-, Darm-, Nasen-, Mund-, Rachen- oder Augenschleimhaut mit dem permeabilitätssteigernden Copolymer zu behandeln und anschließend den pharmazeutischen Wirkstoff zu verabreichen.

Die oben genannten pharmazeutischen Zubereitungen werden in der Regel unter Zusatz von Füll-, Binde-, Spreng-, Gleitmitteln oder anderen Hilfsstoffen hergestellt. Als Füllstoffe und Trockenbindemittel für Tabletten dienen u.a. Lactose, Saccharose, Mannit, Sorbit, mikrokristalline Cellulose, Stärke, Dicalciumphosphat und Polyglycole. Geeignete Bindemittel für die Granulierung sind Stärke, Alginate, Polyvinylpyrrolidon und besonders Carboxymethylcellulose. Geeignete Gleitmittel sind z.B. Stärke, Talkum und Siliciumdioxid. Als Schmiermittel bei der maschinellen Tablettenherstellung sind Magnesiumstearat sowie Metallseifen brauchbar. Zu den gebräuchlichen Tablettensprengmittel zählen u.a. Stärke, Cellulosederivate, Alginate, Dextrane sowie quervernetztes Polyvinylpyrrolidon.

Je nach Anwendungsgebiet und Wirkstoff werden die Copolymere vorteilhafterweise in neutralisierter, teilneutralisierter oder in nichtneutralisierter Form verwendet. Liegen die Copolymere in nichtneutralisierter Form vor, so ist die Anwesenheit einer Base oder eines Protonenakzeptors, der entweder aus einem weiteren Hilfsstoff und/oder direkt aus dem Wirkstoff besteht, oftmals vorteilhaft.

Im Falle eines basischen Wirkstoffs kann dieser ganz oder teilweise in Salzform mit dem erfindungsgemäßen Copolymer aus a), b) und ggf. c) vorliegen.

Die nachfolgenden Beispiele veranschaulichen die erfindungsgemäße Verwendung der (Meth)acrylsäure:Maleinsäure Copolymere, wobei deren permeabilitätssteigernde Wirkung in einem in vitro Testsystem am Beispiel humaner Darmepithelzellen (Caco-2-Zellkulturen) nachgewiesen wurde. Caco-2-Zellen besitzen viele Differenzierungseigenschaften von Epithelzellen, wie die asymmetrische Verteilung von Enzymen, morphologisch ähnlicher Aufbau mit Microvilli auf der apikalen Seite der Zellen und Ausbildung eines Monolayers mit tight-junctions. Wenn diese Zellen auf poröse Membranen in sogenannten transwell-Platten kultiviert werden, kann man exakt den Transport vom apikalen Kompartiment (Darmlumen) ins basolaterale Kompartiment (Lymphe) untersuchen. Dabei zeigen sowohl der transepitheliale elektrische Widerstand (TEER) als auch die Permeation radioaktiver Marker (¹⁴C-Mannitol) durch die Zell-Monoschicht an, inwieweit die tight junctions geöffnet bzw. geschlossen sind. Eine reversible Permeabilitätsverbesserung läßt sich bei Messungen des TEER's am Wiederansteigen des Widerstandwertes nach Austausch des apikalen Testmediums mit dem ursprünglichen apikalen Medium erkennen.

### Versuchsbeispiele:

### 11. Bestimmung des transepithelialen Widerstandes (TEER):

Auf Polycarbonat-Filter (0 6.5 mm) kultivierte Caco-2-Zellen wurden in Costar Transwell-Kammern (Costar Europe Ltd., Badhoevedorp, NL) bis zu einer Zelldichte von 10⁴ Zellen/cm² herangezüchtet. Als Kulturmedium wurde in beiden Halbzellen DMEM (Dulbecco's Modified Eagle's Medium, Sigma) unter Zusatz von 1 % einer nichtessentiellen Aminosäurelösung, 10 % fötalem Rinderserum, Benzyl-penicillin G (160 U/ml) und Streptomycin Sulfat (100 µg/ml) verwendet, wobei das Medium täglich ausgetauscht wurde. Die Kulturplatten wurden bei 37°C in einer Atmosphäre aus 95 % Luft und 5 % CO₂ inkubiert.

Die zu untersuchenden Polymere (siehe Tabelle 1) wurden in DMEM in unterschiedlichen Konzentrationen (1 - 7.5 %) gelöst und mit NaOH neutralisiert. Kontrollversuche wurden in DMEM durchgeführt. Der transepitheliale Widerstand (TEER) der einzelnen Filter wurde nach Inkubation im Abstand von 20 Minuten mit einem Millicell-Electrical-Resistance-System der Fa. Millipore B.V.(Etten-Leur, NL) gemessen. Zur Überprüfung der Reversibilität der Zell-Zell Wechselwirkung wurde nach 2h die Polymer-Lösung gegen reines DMEM ausgetauscht und der Widerstand erneut gemessen.

**Tabelle 1:**

| Struktur der untersuchten Polymere | | |
|---|---|---|
| Polymer | Struktur | mittleres Molekulargewicht |
| 1 | AS/MS = 70/30 | 70000 |
| 2 | AS/MS = 70/30 | 150000 |
| 3 (Vergleich) | AS = 100 | 250000 |
| 4 | AS/MS/HPA = 40/40/20 | 20000 |
| 5 | AS/MS = 50/50 | 50000 |
| 6 | AS/MS/VS = 35/35/30 | 15000 |
| Abkürzungen: | | |
| AS: Acrylsäure | | |
| MS: Maleinsäure | | |
| HPA: Hydroxypropylacrylat | | |
| VS: Vinylsulfonsäure | | |

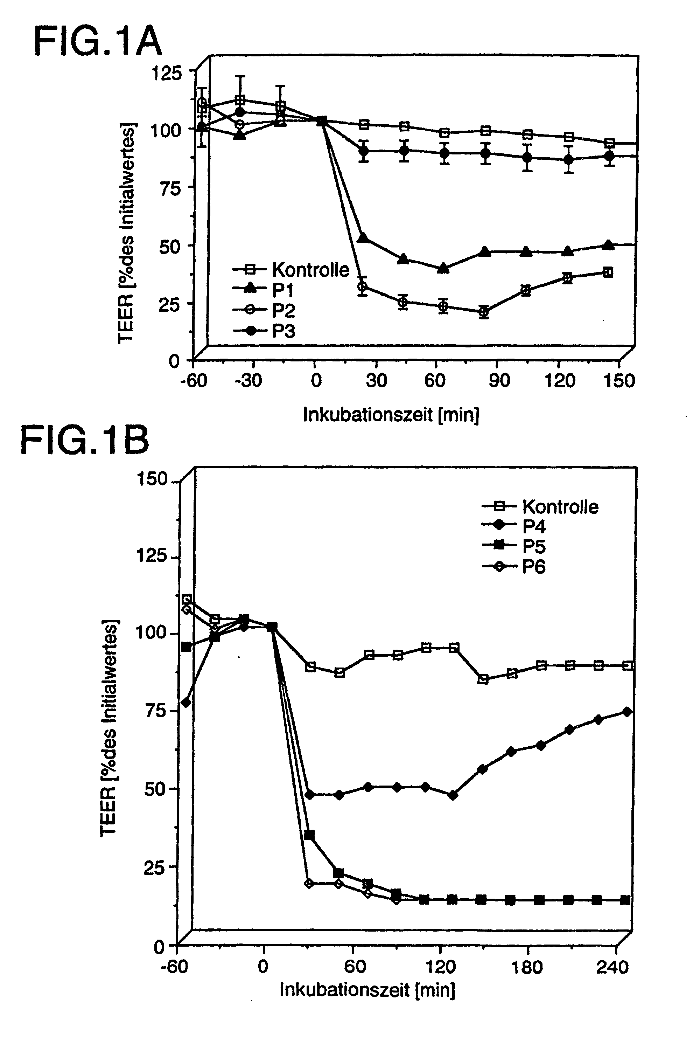

In Figur 1 A/B sind die Versuchsergebnisse mit den Polymeren 1 bis 6 (5%ige Lösung in DMEM) wiedergegeben. Es zeigt sich, daß der transepitheliale elektrische Widerstand bei Verwendung der Copolymere 1, 2 sowie 4 bis 6 deutlich abfällt, während mit dem Vergleichspolymer 3 nur eine geringe Absenkung zu beobachten ist.

### 12. Bestimmung der ¹⁴C-Mannitol-Penetration:

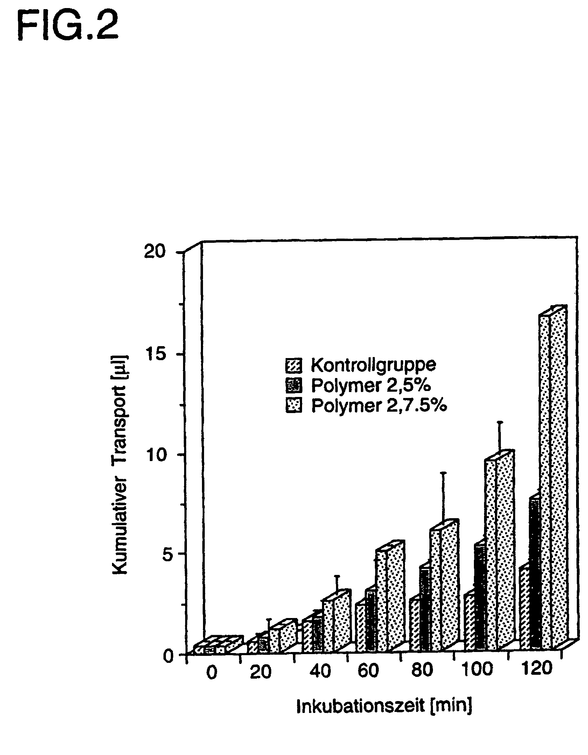

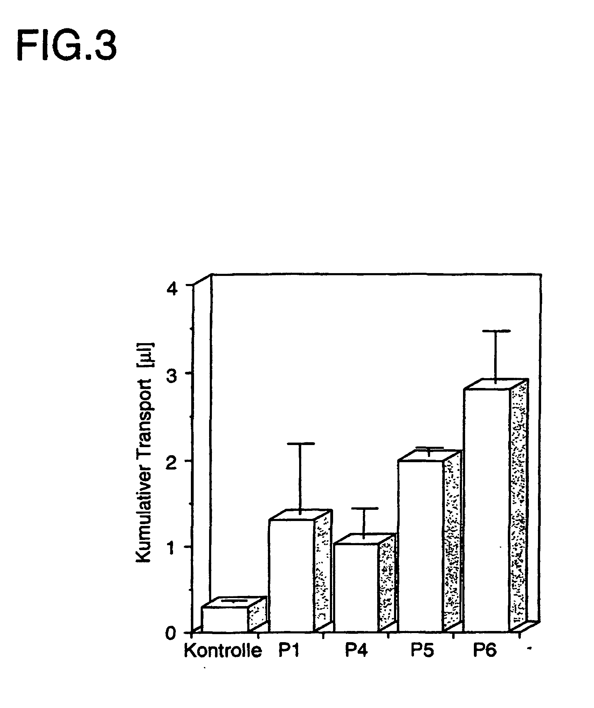

Die Erhöhung des parazellulären Transportes wurde durch Inkubation der zellulären Monoschicht mit einem geeigneten hydrophilen Marker (¹⁴C-Mannitol, 4 µmol/l, spez. Aktivität: 0.2 µCi/ml) in Gegenwart einer 5 bzw. 7.5%igen Polymerlösung untersucht. Nach jeweils 30 Minuten wurden 200 µl Proben aus der Akzeptor-Kammer entnommen und auf ihre Radioaktivität überprüft (Tri-carb 1500 scintillation counter, Packard Instr. B.V., Groningen, NL). Wie die Figuren 2 (5 bzw. 7.5%ige Lösung von Polymer 2 in DMEM) und 3 (5%ige Lösung der Polymere 1, 4-6 in DMEM, Inkubationszeit 120 min) zeigen, führt die Inkubation von Caco-2-Zellen in Gegenwart der untersuchten Copolymere 1, 2 sowie 4 bis 6 zu einer Öffnung der tight junctions zwischen den Epithelialzellen und im Vergleich zur Kontrolle zu einer signifikanten Erhöhung der Transportrate des radioaktiven Markers.

### 13. Beispiele für pharmazeutische Darreichungsformen.

### a. Atenolol-Tabletten

| | |
|---|---|
| Atenolol | 50 mg |
| Ludipress® | 50 mg |
| Acrylsäure-Maleinsäure-Copolymer (50:50)-Natriumsalz (Molekulargewicht 50 000) | 146,5 mg |
| Aerosil® 200 | 2 mg |
| Kollidon® VA64 | 1 mg |
| Magnesiumstearat | 1,5 mg |
| Tablettenmasse | 260 mg |

1000 g Atenolol, 1000 g Ludipress® (BASF), 2930 g Acrylsäure-Maleinsäure-Copolymer (50:50)- Natriumsalz , 40 g Aerosil® 200 (Degussa), 200 g Kollidon® VA64 (BASF) und 30 g Magnesiumstearat wurden über ein 0,8 mm Sieb vorgesiebt und in einem Turbula-Mischer 5 min gemischt. Anschließend wurden auf einer Rundläufertablettenpresse bei einer Preßkraft von 22 kN gewölbte Tabletten mit einem Durchmesser von 9 mm hergestellt.

### b. Sulfasalazin-Filmtabletten

| | |
|---|---|
| Sulfasalazin | 250 mg |
| Acrylsäure-Maleinsäure-Copolymer (70:30)-Natriumsalz (Molekulargewicht 70 000) | 500 mg |
| Kollidon® 30 | 15 mg |
| Aerosil® 200 | 2 mg |
| Magnesiumstearat | 3 mg |
| | 770 mg |

500 g Sulfasalazin und 1000 g Acrylsäure-Maleinsäure-Copolymer (70:30)-Natriumsalz wurden in einem Stephan-Mischer 2 min gemischt und unter Rühren mit einer Lösung von 30 g Kollidon® 30 in 230 g Isopropanol versetzt. Die feuchte Masse wurde durch ein Sieb mit einer Maschenweite von 1,0 mm gegeben und auf einer Horde bei Raumtemperatur abgetrocknet. Nach der Siebung des trockenen Materials über ein 1,0 mm-Sieb wurden 4,0 g Aerosil 200 und 6,0 g Magnesiumstearat zugesetzt und 5 min. gemischt. Die Pulvermischung wurde anschließend auf einer Rundläuferpresse bei einer Preßkraft von 35 kN und einer Umdrehungsgeschwindigkeit von 30 U/min zu Oblongtabletten im Footballshape-Format mit den Ausmaßen 19 x 8,5 mm verpreßt.

In einem 2. Schritt wurden die Oblongtabletten in einem Horizontaltrommelcoater mit einem magensaftresistenten Filmüberzug versehen. Die bei einer Zuluft von 50°C aufzutragende Sprühdispersion wies folgende Zusammensetzung auf:

| | |
|---|---|
| Titandioxid | 0,5 % |
| Talkum | 2 % |
| Sicovit® Rot 30 | 0,5 % |
| Kollidon® 30 | 0,5 % |
| Methacrylat-Ethylacrylat Copolymer (1:1) | 15 % |
| Triethylcitrat | 1,5 % |
| Wasser | 80 % |

Von dieser Dispersion wurden pro Tablette 100 mg entsprechend 15 mg Methacrylsäure-Ethylacrylat-Copolymer aufgetragen.

### c. Furosemid-Mikrotabletten

| | |
|---|---|
| Furosemid | 1 mg |
| Acrylsäure-Maleinsäure-Copolymer (70:30)-Natriumsalz | 5,7 mg |
| Kollidon® VA64 | 0,2 mg |
| Aerosil® 200 | 0,05 mg |
| Magnesiumstearat | 0,05 mg |
| Tablettenmasse | 7,0 mg |

100 g Furosemid, 570 g Acrylsäure-Maleinsäure-Copolymer (70:30)-Natriumsalz und 20 g Kollidon® VA64 wurden in einem Stephan-Mischer gemischt und unter Rühren mit 105 g Isopropanol befeuchtet. Die feuchte Masse wurde durch ein Sieb mit einer Maschenweite von 0,6 mm gedrückt und auf einer Horde in dünner Schicht 24 h bei Raumtemperatur getrocknet. Das trockene Granulat wurde über ein 0,8 mm-Sieb gegeben mit ebenfalls gesiebtem Magnesiumstearat und Aerosil® 200 versetzt und im Turbula-Mischer 5 min. gemischt und nochmals über ein 0,8 mm-Sieb gegeben. Auf einer Excenterpresse des Typs Korsch EKO wurden danach gewölbte Mikrotabletten mit 2 mm Durchmesser und ca. 2 mm Höhe hergestellt. Um eine Dosierung von 40 mg pro abgeteilte Form zu erreichen, wurden jeweils 40 Mikrotabletten in Gelatinesteckkapseln gefüllt.

### d. Methyldopa-Tabletten

| | |
|---|---|
| Methyldopa | 250 mg |
| Acrylsäure-Maleinsäure-Copolymer (50:50)-Natriumsalz (Molekulargewicht 50 000) | 347,5 mg |
| Aerosil® 200 | 3,5 mg |
| Kollidon® VA64 | 26 mg |
| Magnesiumstearat | 3 mg |
| | 630 mg |

2500 g Methyldopa, 3475 g Acrylsäure-Maleinsäure-Copolymer (50:50)-Natriumsalz, 35 g Aerosil 200, 260 g Kollidon® VA64 und 30 g Magnesiumstearat wurden über ein 0,8 mm-Sieb vorgesiebt und in einem Turbulamischer 5 min gemischt. Anschließend wurde diese Pulvermischung auf einer Rundläufertablettenpresse bei einer Preßkraft von 30 kN und einer Geschwindigkeit von 30 Umdrehungen/min zu biplanen, facettierten Tabletten mit einem Durchmesser von 12 mm verpreßt.

### e. S-Adenosylmethionin-Lutschtabletten

| | |
|---|---|
| S-Adenosylmethionin | 100 mg |
| Acrylsäure-Maleinsäure-Copolymer (50:50)-Natriumsalz | 700 mg |
| Mannit | 200 mg |
| Aspartame | 3 mg |
| Orangenaroma | 5 mg |
| Kollidon® VA 64 | 82 mg |
| Aerosil® 200 | 5 mg |
| Magnesiumstearat | 5 mg |
| Tablettenmasse | 1100 mg |

500 g S-Adenosylmethionin, 3500 g Acrylsäure-Maleinsäure-Copolymer (50:50)-Natriumsalz, 1000 g Mannit, 15 g Aspartame, 25 g Orangenaroma und 410 g Kollidon® VA 64 wurden über ein 0,8 mm-Sieb vorgesiebt und im Turbula-Mischer 5 min gemischt. Anschließend wurden 25 g Magnesiumstearat und 25 g Aerosil® 200, die zuvor über ein 0,5 mm-Sieb gesiebt wurden, zugegeben und in 2,5 min untergemischt. Auf einer Rundläufertablettenpresse wurden bei einem Preßdruck von 35 kN und einer Umdrehungsgeschwindigkeit von 30 U/min biplane, facettierte Tabletten mit 1100 mg Gewicht hergestellt.

### f. Cefuroxim-Granulat

| | |
|---|---|
| Cefuroximaxetil (entsprechend 250 mg Cefuroxim) | 300,7 mg |
| Acrylsäure-Maleinsäure-Copolymer (50:50)-Natriumsalz (Molekulargewicht 50 000) | 1000 mg |
| Methacrylsäure-Methylmethacrylat-Copolymer (1:1) | 150 mg |
| Saccharose | 502,3 mg |
| Orangenaroma | 5 mg |
| Aspartame | 2 mg |
| Kollidon® VA 64 | 40 mg |
| | 2000 mg |

300,7 g Cefuroximaxetil (entsprechend 250 g Cefuroxim), 1000 g Acrylsäure-Maleinsäure-Copolymer (50:50)-Natriumsalz, 150 g Methacrylsäure-Methylmethacrylat-Copolymer (1:1), 502,3 g Saccharose, 5 g Orangenaroma und 2 g Aspartame wurden in einem Stephan-Mischer gemischt und unter Rühren mit einer Lösung von 40 g Kollidon® VA 64 in 310 g Isopropanol befeuchtet. Die feuchte Masse wurde durch ein Sieb mit einer Maschenweite von 1,2 mm gedrückt und bei Raumtemperatur über 24 h auf einer Horde langsam abgetrocknet. Das trockene Granulat wurde in Siegelrandbeutel mit einer Dosierung von 2000 mg abgefüllt.

### g. Gonadorelin-Nasenspray

| | |
|---|---|
| Gonadorelin | 1,0 mg |
| Benzylalkohol | 20 mg |
| Acrylsäure-Maleinsäure-Copolymer (50:50)-Natriumsalz (Molekulargewicht 50 000) | 75 mg |
| 1-n-HCl quantum satis pH 7,0 | |
| Wasser | ad 1000 mg |

700 g gereinigtes, keimfreies Wasser wurden vorgelegt und mit 20 g Benzylalkohol versetzt. Anschließend wurden 50 g Acrylsäure-Maleinsäure-Copolymer darin gelöst und der pH-Wert mit 1-n-HCl unter Rühren auf pH 7,0 gebracht. Nach der Zugabe von Gonadorelin wurde noch 0,5 h gerührt, die restliche Wassermenge ad 1 000 g ergänzt die Lösung durch ein 0,22 µm Filter sterilfiltriert und in Nasensprayflaschen abgefüllt.

### h. Ranitidin-Lösung

| | |
|---|---|
| Ranitidin-HCl | 20 mg |
| Acrylsäure-Maleinsäure-Copolymer (50:50)-Natriumsalz (Molekulargewicht 50 000) | 100 mg |
| Saccharose | 50 mg |
| Benzalkoniumchlorid | 1 mg |
| 1-n-HCl | 54 mg |
| Gereinigtes Wasser | 775 mg |
| | 1000 mg |

20 g Ranitidin-HCl, 50 g Saccharose und 1 g Benzalkoniumchlorid wurden in 775 g gereinigtes Wasser unter Rühren gelöst. Nach Zugabe von 100 g Acrylsäure-Maleinsäure-Copolymer (50:50)-Natriumsalz und 54 g 1-n-Salzsäure wurde bis zum vollständigen Auflösen weitergerührt, die Lösung durch ein 5 µm-Filter filtriert und in 50 g-Flaschen abgefüllt.

## Patentansprüche

1. Verwendung von (Meth)acrylsäure-Maleinsäure-Copolymeren zur Herstellung von Arzweimitteln Verbesserung der Permeabilität der Schleimhaut.

2. Verwendung von Copolymeren nach Anspruch 1, enthaltend
a) 10 bis 90 mol-% (Meth)acrylsäure,
b) 90 bis 10 mol-% Maleinsäure und
c) gegebenenfalls 0 bis 40 mol-% weiterer Monomere.

3. Verwendung nach den Ansprüchen 1 und 2 **dadurch gekennzeichnet, daß** als weitere Monomere c) bis zu 40 mol-% Hydroxyalkyl(meth)acrylat, (Meth)acrylsäurealkylester und/oder Vinylsulfonsäure im Copolymer enthalten sind.

4. Verwendung nach den Ansprüchen 1 und 2 **dadurch gekennzeichnet, daß** das (Meth)acrylsäure:Maleinsäure-Molverhältnis von 70:30 bis 30:70 variiert.

5. Die Permeabilität der Schleimhaut verbessernde pharmazeutische Zubereitungen enthaltend (Meth)acrylsäure-Maleinsäure-Copolymere gemäß den Ansprüchen 1 bis 4.

6. Pharmazeutische Zubereitungen nach Anspruch 5 in Form von Tabletten, Extrudaten, Granulaten, Pellets, Pulver, Kapseln, Suppositorien, Salben, Suspensionen, Lösungen oder Emulsionen.

7. Pharmazeutische Zubereitungen nach den Ansprüchen 5 und 6 mit einem (Meth)acrylsäure-Maleinsäure-Copolymer-Anteil größer 10 Gew.-% der Gesamtmasse.

8. Pharmazeutische Zubereitungen nach den Ansprüchen 5 bis 7, in der die Copolymere in nichtneutralisierter, teilneutralisierter oder neutralisierter Form vorliegen, wobei im Fall der nichtneutralisierten Form die Zubereitung zusätzlich eine Base oder einen Protonenakzeptor enthalten kann.

9. Pharmazeutische Zubereitungen nach den Ansprüchen 5 bis 8, in der der Wirkstoff ganz oder teilweise in Salzform mit dem (Meth)acrylsäure-Maleinsäure-Copolymer vorliegt.

10. Verwendung der pharmazeutischen Zubereitungen gemäß den Ansprüchen 5 bis 9 zur Herstellung von Arzweimitteln zur oralen, sublingualen, buccalen, rectalen, pulmonalen oder nasalen Applikation oder zur Applikation über die Augenschleimhaut.

## Claims

1. The use of (meth)acrylic acid/maleic acid copolymers for producing medicaments for improving mucosal permeability.

2. The use of copolymers as claimed in claim 1, comprising
a) 10-90 mol% of (meth)acrylic acid,
b) 90-10 mol% of maleic acid and
c) with or without 0-40 mol% of other monomers.

3. The use as claimed in claims 1 and 2, wherein the other monomers c) present are up to 40 mol% of hydroxyalkyl (meth)acrylate, alkyl (meth)acrylate and/or vinylsulfonic acid in the copolymer.

4. The use as claimed in claims 1 and 2, wherein the (meth)acrylic acid:maleic acid molar ratio varies from 70:30 to 30:70.

5. A pharmaceutical composition improving mucosal permeability, comprising (meth)acrylic acid/maleic acid copolymers as set forth in claims 1 to 4.

6. A pharmaceutical composition as claimed in claim 5 in the form of tablets, extrudates, granules, pellets, powders, capsules, suppositories, ointments, suspensions, solutions or emulsions.

7. A pharmaceutical composition as claimed in claims 5 and 6 with a (meth)acrylic acid/maleic acid copolymer content of more than 10% of the total weight.

8. A pharmaceutical composition as claimed in any of claims 5 to 7, in which the copolymers are present in unneutralized, partially neutralized or neutralized form, and in the case of the unneutralized form the composition may additionally comprise a base or a proton acceptor.

9. A pharmaceutical composition as claimed in any of claims 5 to 8, in which the active ingredient is present wholly or partly in the form of a salt with the (meth)acrylic acid/maleic acid copolymer.

10. The use of the pharmaceutical compositions as claimed in any of claims 5 to 9 for producing medicaments for oral, sublingual, buccal, rectal, pulmonary or nasal administration or for administration through the mucosa of the eye.

## Revendications

1. Utilisation de copolymères d'acide (méth)acrylique-acide maléique pour la préparation de médicaments pour l'amélioration de la perméabilité des muqueuses.

2. Utilisation de copolymères selon la revendication 1, contenant
a) 10 à 90 % en mol d'acide (méth)acrylique,
b) 90 à 10 % en mol d'acide maléique et
c) éventuellement 0 à 40 % en mol d'autres monomères.

3. Utilisation selon les revendications 1 et 2, **caractérisée par le fait que** sont contenus comme autres monomères c) jusqu'à 40 % en mol de (méth)acrylate d'hydroxyalkyle, d'ester alkylique d'acide (méth)acrylique et/ou d'acide vinylsulfonique dans le copolymère.

4. Utilisation selon les revendications 1 et 2, **caractérisée par le fait que** le rapport molaire acide (méth)acrylique:acide maléique varie de 70:30 à 30:70.

5. Compositions pharmaceutiques améliorant la perméabilité des muqueuses, contenant des copolymères d'acide (méth)acrylique-acide maléique selon les revendications 1 à 4.

6. Compositions pharmaceutiques selon la revendication 5, sous forme de comprimés, produits d'extrusion, granulés, pellets, poudres, capsules, suppositoires, baumes, suspensions, solutions ou émulsions.

7. Compositions pharmaceutiques selon les revendications 5 et 6, avec une fraction de copolymère d'acide (méth)acrylique-acide maléique supérieure à 10 % en poids de la masse totale.

8. Compositions pharmaceutiques selon les revendications 5 à 7, dans lesquelles les copolymères se présentent sous forme non-neutralisée, partiellement neutralisée ou neutralisée, tandis que dans le cas de la forme non-neutralisée la composition peut contenir en outre une base ou un accepteur de protons.

9. Compositions pharmaceutiques selon les revendications 5 à 8, dans lesquelles la substance active se présente en totalité ou en partie sous forme de sel avec le copolymère d'acide (méth)acrylique-acide maléique.

10. Utilisation des compositions pharmaceutiques selon les revendications 5 à 9 pour la préparation de médicaments pour application orale, sublinguale, buccale, rectale, pulmonaire ou nasale, ou pour application par l'intermédiaire de la muqueuse oculaire.
